(19) Europäisches Patentamt
European Patent Office
Office européen des brevets

(11) **EP 4 226 910 A1**

(12) **EUROPÄISCHE PATENTANMELDUNG**

(43) Veröffentlichungstag:
**16.08.2023 Patentblatt 2023/33**

(21) Anmeldenummer: **22211454.8**

(22) Anmeldetag: **05.12.2022**

(51) Internationale Patentklassifikation (IPC):
**A61K 8/37** (2006.01)          **A61K 8/49** (2006.01)
**A61K 8/55** (2006.01)          **A61Q 5/12** (2006.01)

(52) Gemeinsame Patentklassifikation (CPC):
**A61Q 5/12; A61K 8/37; A61K 8/4973; A61K 8/55;**
A61K 2800/30; A61K 2800/34; A61K 2800/5922;
A61K 2800/596

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(30) Priorität: **31.01.2022 DE 102022200984**

(71) Anmelder: **Beiersdorf AG
20245 Hamburg (DE)**

(72) Erfinder:
• **Reiter, Katharina
21357 Barum (DE)**
• **Nemnich, Julia
22589 Hamburg (DE)**

(74) Vertreter: **Beiersdorf AG
Patentabteilung
Unnastraße 48
20245 Hamburg (DE)**

(54) **EMULSION ZUR HAARPFLEGE UND ZUM STYLEN DER HAARE**

(57) Emulsion, insbesondere eine Emulsion vorgesehen für eine Haar-kosmetische Anwendung, weiter insbesondere ein Creme Gel, die gleichzeitig das Haar pflegt und stylt, wobei die Emulsion kein PEG und keine PEG-haltigen Verbindungen, keine Silikonverbindungen, keine Verbindungen fossilen Ursprungs und keine Verbindungen, die Komponenten enthalten, die fossilen Ursprungs sind und keine synthetischen Polymeren enthält.

**Beschreibung**

Die vorliegende Erfindung bezieht sich auf eine Emulsion, die auf das Haar aufgetragen werden kann und gleichzeitig das Haar pflegen und stylen kann. Die genannte Emulsion ist eine kosmetische Zubereitung und zur Anwendung auf menschlichem Kopfhaar geeignet und insbesondere dafür vorgesehen.

Eine derartige Emulsion kann in Form eines Creme Gels oder Cream Gels vorliegen. Solche Zubereitungen werden in der Regel aus einem Tiegel entnommen und auf dem Haar verteilt. Diese Produkte haben dann üblicherweise einen pflegenden Effekt für die Haare und bewirken auch ein Styling, beides in einem Anwendungsschritt. Beim Verbraucher sind diese Produkte sehr beliebt, denn mithilfe dieser Produkte können die Haare gepflegt werden, insbesondere in Bezug auf die Geschmeidigkeit. Ebenso kann auch eine Regeneration der Haare erzielt werden, indem der Haaroberfläche Komponenten zugeführt werden, die Spliss und Haarrauigkeit entgegenwirken. All diese Eigenschaften tragen dazu bei, dass diese Emulsionsprodukte sehr beliebt sind und vom Verbraucher gerne angewendet werden.

Eine Auswahl von Creme Gelen, die im StdT offenbart wurden, wird im Folgenden beschrieben: Ein "Styling Cream Gel" von der Firma Beiersdorf ist seit September 2020 erhältlich (Mintel-Eintragungsnummer 8097745). Gemäß INCI-Liste enthält dieses Produkt u.a. das synthetische Styling-Polymer PVP (Polyvinylpyrrolidone), Dimethicone (ein Silikonöl) und PEG-90M (ein Polymer aus Ethylenglycol-Resten).

Seit Mai 2020 ist ein "Normal Hold Pure Style Cream Gel" der Firmen Schwarzkopf & Henkel erhältlich (Mintel-Eintragsnummer: 7253517). Laut INCI-Liste enthält dieses Produkt die synthetischen Polymere PVP und Natrium Acrylate/Acryloyldimethyltaurate/Dimethylacrylamide Crosspolymer, sowie dem Emulgator PEG-100 Stearate, der wie der Name anzeigt einen Polyethylenglycol-Anteil aufweist.

Auf der Verpackung des Creme Gels "Light Hold Pure Style Cream Gel" (Mintel Eintragsnummer: 7253517), ebenfalls von den Firmen Schwarzkopf & Henkel, wird ausgelobt, dass u.a. keine Silikone enthalten sind. Enthalten sind aber die synthetischen Polymere PVP und Natrium Acrylate/Acryloyldimethyltaurate/Dimethylacrylamide Crosspolymer, sowie der Emulgator PEG-100 Stearate.

Von der Firma L'Oréal ist seit Februar 2020 das Creme Gel "Texturinsing Gel Cream Beach Effect" erhältlich (Mintel Eintragsnummer 7245421). Gemäß INCI-Liste sind die synthetischen Polymere PEG-150/decyl alcohol/SMDI Copolymer und Polyquaternium-37, sowie die PEG-haltigen Verbindungen PEG-100 Stearate und PPG-1 Trideceth-6 enthalten.

Alle diese Produkte enthalten synthetische Polymere und/oder Silikonverbindungen und/oder PEG-haltige Komponenten.

Die erfindungsgemäße Emulsion ist vorteilhaft zur Haarpflege und zum Haar-Styling geeignet. Insbesondere vorteilhaft ist die genannte Emulsion als Creme Gel ausgestaltet. Die erfindungsgemäßen Emulsionen sind insbesondere O/W-Emulsionen.

Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Die Flüssigkeiten (rein oder als Lösungen) liegen in einer Emulsion in einer mehr oder weniger feinen Verteilung vor, die im Allgemeinen nur begrenzt stabil ist.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter, zum Beispiel elektrische Leitfähigkeit, Sensorik, Anfärbbarkeit der kontinuierlichen Phase, einer O/W-Emulsion ist durch das Wasser geprägt. Bei einer Wasser-in-Öl-Emulsion (W/O-Emulsion, z.B. Butter) handelt es sich um das umgekehrte Prinzip, wobei der Grundcharakter hier durch das Öl bestimmt wird.

Emulsionen benötigen zu ihrer Bildung und zur Stabilisierung im Allgemeinen einen oder mehrere Emulgatoren, gegebenenfalls auch Verdicker und/oder Konsistenzgeber, um über einen kosmetisch akzeptablen Zeitraum stabil zu sein.

Emulgatoren bewirken, dass die zwei nicht miteinander mischbaren Flüssigkeiten (zum Beispiel Öl in Wasser) sich zu einer Emulsion vermengen lassen. Aufgrund des amphiphilen Charakters dringen sie mit ihrem fettlöslichen Teil in das Öl ein. Durch den hydrophilen Teil können die durch Rühren oder Homogenisieren entstandenen Öltröpfchen in der wässrigen Umgebung dispergiert werden. Emulgatoren haben primär keinen waschaktiven, tensidischen Charakter.

Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe). Dabei kann zwischen nicht-ionischen, anionischen und kationischen Emulgatoren unterschieden werden.

Ein Kennzeichen für die Hydrophilie eines gegebenen Emulgators ist dessen HLB-Wert, der sich nach folgender Formel ergibt:

$$HLB = 20 \times (1- M_{lipophil}/M),$$

wobei $M_{lipophil}$ für die Molmasse des lipophilen Anteils im Emulgator und M für die Molmasse des gesamten Emulgators steht.

Im Allgemeinen gelten Emulgatoren mit einem HLB-Wert bis ca. 8 als W/O-Emulgatoren. O/W Emulgatoren hingegen weisen HLB-Werte von größer 8 bis etwa 15 auf. Substanzen mit HLB Werten größer 15 werden häufig als Lösungsvermittler bezeichnet.

Die üblicherweise eingesetzten Emulgatoren oder emulgierend wirkenden Substanzen zeichnen sich oftmals durch das Vorhandensein von Polyethylenglycol-Anteilen im Molekül aus. Polyethylenglycol wird häufig mit PEG abgekürzt, die Polyethylenglycol-Anteile werden oftmals kurz als PEGs bezeichnet. Polyethylenglycol-Anteile bestehen aus mindestens zwei mit einander verknüpften Ethylenglycolmolekülen, es können auch Hunderte von Ethylenglycolmolekülen sein, beispielsweise 200. Inhaltsstoffe, die diese Polyethylenglycol-Anteile enthalten sind beim Verbraucher zunehmend unerwünscht. Es war somit auch die Aufgabe der vorliegenden Erfindung, Inhaltsstoffe mit Polyethylenglycol-Anteilen nicht in die erfindungsgemäßen Zubereitungen einzuarbeiten und stattdessen Inhaltsstoffe zu identifizieren, die, bei ihrer Einarbeitung, die Produktleistungen hinsichtlich Pflege und Styling nicht negativ beeinflussen.

Emulsionen, die für die Haar-kosmetische Anwendung vorgesehen sind, können die Haare pflegen. Zu diesem Zweck enthalten sie Komponenten, die zur Pflege des menschlichen Haares besonders gut geeignet sind. Diese Substanzen treten mit der äußeren Schicht des Haares, der Cuticula in Wechselwirkung.

Die Cuticula wird auch äußere Schuppenschicht genannt und besteht aus dachziegelartig einander überlappenden Hornschüppchen, die über der Haarrinde (Cortex) liegen. Die 4 bis 5 $\mu$m dicke Cuticula schützt das Haar vor physikalischen und chemischen Beeinflussungen (Licht, kosmetische Behandlung etc.). Sie kann in eine stark Cysteinhaltige (25 - 30 %), Proteolyse-stabile Exocuticula und eine Proteolyse-empfindliche Endocuticula unterteilt werden. Beim gesunden Haar liegt die Schuppenschicht flach an und ergibt so eine glatte, durchscheinende Oberfläche. Das Licht wird optimal reflektiert und ergibt so den gesunden Glanz des Haares.

Die Cuticula ist von einer Fettschicht überzogen, die Triglyceride, freie Fettsäuren, Wachse, Squalene und zu einem geringen Teil auch Cholesterin enthält. Darüber hinaus ist 18-Methyl-Eicosansäure mit der äußeren Oberfläche der Cuticula-Schuppen kovalent verknüpft. Diese Substanz trägt wesentlich zur Hydrophobizität der Haaroberfläche bei.

Mit den Emulsionen, insbesondere Emulsionen für eine Haar-kosmetische Anwendung, können dem Haar lipophile und Feuchtigkeits-spendende Komponenten zurückgegeben werden, die beispielsweise durch Behandlungen des Haares, wie z.B. häufige Haarwäschen, und Umwelteinflüsse verringert wurden. Deshalb sind im allgemeinen lipophile Komponenten in derartigen Zubereitungen enthalten, die mit den lipophilen Verbindungen der Cuticula wechselwirken können.

Neben der Hydrophobizität der Cuticula, ist auch die Proteinstruktur der Cuticula zu beachten. Die Zusammensetzung der Proteinstruktur resultiert in einem isoelektrischen Punkt von etwa 3,67 an der Haaroberfläche. Der pH-Wert, den die üblicherweise eingesetzten Haar-kosmetische Zubereitungen aufweisen, bewirkt, dass negativ geladene Strukturen auf der Haaroberfläche vorliegen.

Pflegende Komponenten in Emulsionen, die zur Haar-kosmetischen Anwendung vorgesehen sind, können also lipophile Verbindungen, die mit den lipophilen Verbindungen an der Cuticula-Oberfläche in Wechselwirkung treten, und/oder positive geladene Verbindungen (kationische Verbindungen), die mit den negative geladenen Strukturen auf der Haaroberfläche wechselwirken, umfassen.

In der Vergangenheit wurden vielfach Silikon-basierte Verbindungen und/oder Mineralöle als Pflegekomponenten in Haarpflege-Zubereitungen eingesetzt. Silikone können als Silikonöle vorliegen, die hydrophob sind und mit den lipophilen Komponenten auf der Haaroberfläche wechselwirken und idealerweise einen Schutzfilm auf der Haaroberfläche ausbilden.

Silikonverbindungen können substituiert sein, und zwar mit chemischen Gruppen, die positive Ladungen aufweisen (kationische Silikonverbindungen). Diese Silikonverbindungen können auch mit den negativ geladenen Strukturen an der Haaroberfläche in Wechselwirkung treten.

Neben Silikonverbindungen werden vielfach auch kationische Polymere als pflegende Komponenten eingearbeitet. Hierbei handelt es sich in der Regel um Polymere, die zumeist synthetischen Ursprungs sind. Diese kationische Pflegepolymere können ebenfalls mit den negativ geladenen Oberflächenstrukturen des Haares wechselwirken.

Der Einsatz der oben genannten großen Klassen von Pflegekomponenten kann jedoch auch Nachteile haben. Teilweise leidet die Textur dieser Zubereitungen; sie lassen sich dann nur schlecht im Haar verteilen. Ein weiterer Nachteil, der oft bei Silikon-haltigen Zubereitungen beobachtet wird, ist es, dass die Haare "beschwert" sind, d.h. der Film auf den Haaren wurde bei der Haarwäsche nicht vollständig weggewaschen und baute sich immer weiter auf. Eine Frisurgestaltung ist dann überaus schwierig.

Als weiterer Aspekt ist auch das zunehmende Umweltbewusstsein zu nennen. Der Verbraucher wünscht sich Zubereitungen, die natürliche Inhaltsstoffe enthalten und die biologische Abbaubarkeit soll auch gegeben sein. Silikonverbindungen und viele kationische Polymere sind jedoch synthetischer Natur und weisen teilweise eine schlechte biologische Abbaubarkeit auf. Auch die Einarbeitung von Mineralölen ist bei den Verbrauchern zunehmend unerwünscht, weil es sich um Inhaltsstoffe aus fossilen Quellen handelt, die möglicherweise geringe Spuren von aromatischen Mineralölkohlenwasserstoffen (MOAH) enthalten. Es gibt Hinweise, dass sich unter den MOAHs krebserregende Substanzen befinden könnten. Ausgehend von den vorgenannten Ausführungen war es auch Aufgabe der vorliegenden Erfindung, pflegende Komponenten, insbesondere lipophile Komponenten, für Emulsions-Zubereitungen für die Haar-kosmetische Anwendung zu identifizieren, die bei der Einarbeitung in die erfindungsgemäßen Zubereitungen zu stabilen, haptisch ansprechenden Zubereitungen führen, die in der Pflegeleistung die gleichen Effekte erzielen wie herkömmlich Produkte.

Zusammenfassend lautet die Aufgabe der vorliegenden Erfindung somit eine Emulsion, geeignet für die Haar-kosmetische Anwendung, bereit zu stellen, die in Bezug auf die Pflege der Haare wenigstens genauso gut ist, wie Produkte des StdT, dabei aber ohne die herkömmlichen Pflegekomponenten auskommt, die vielfach schlecht biologisch abbaubar und zudem synthetischen oder fossilen Ursprungs sind oder derartige Komponenten enthalten.

Überraschenderweise löst eine Emulsion, insbesondere eine Emulsion vorgesehen für eine Haar-kosmetische Anwendung, weiter insbesondere ein Creme Gel, enthaltend

- eine Kombination von Emulgatoren umfassend wenigstens einen Sorbitanfettsäureester und wenigstens ein Alkylphosphat und
- eine Kombination von wenigstens zwei Emollentien, umfassend wenigstens ein Acylglycerin und wenigstens ein Esteröl, wobei das Esteröl wenigstens 12 Kohlenstoffatome und wenigstens eine Verzweigung aufweist,

wobei die Emulsion frei ist von

- PEG und PEG-haltigen Verbindungen,
- Silikonverbindungen,
- Verbindungen fossilen Ursprungs und Verbindungen, die Komponenten enthalten, die fossilen Ursprungs sind und
- synthetischen Polymeren,

die oben genannten Aufgaben.

Die Emulsion ist eine kosmetische Zubereitung, die insbesondere für eine Haar-kosmetische Anwendung vorgesehen ist und weiter insbesondere ein Creme Gel ist. Die Emulsion ist für die Anwendung auf dem menschlichen Haar geeignet und insbesondere dafür vorgesehen.

Die Emulsion ist eine wässrige Zubereitung; der Wassergehalt beträgt von 50 bis 80 Gew.-%, bevorzugt 55 bis 70 Gew.-%, bezogen auf das Gesamtgewicht der Zubereitung.

Die erfindungsgemäße Emulsion ist derart ausgestaltet, dass eine Entnahme aus einem Tiegel erfolgen kann. Die Emulsion ist insbesondere dafür vorgesehen, dass eine Entnahme aus einem Tiegel erfolgt.

Für eine Entnahme aus einem Tiegel liegt die Konsistenz der erfindungsgemäßen Zubereitung vorteilhaft im Bereich von 20 bis 35 SKT (Skalenteile), gemessen bei 25 °C. Die Bestimmung der Konsistenz erfolgt gemäß der Methode, die im Dokument DE 2909087 C2 beschrieben ist.

Die erfindungsgemäße Emulsion enthält wenigstens einen Sorbitanfettsäureester. Dies sind Ester des Sorbits bzw. des 1,4-Sorbitanhydrids (= Sorbitan). Die Fettsäurereste weisen 6 bis 22 Kohlenstoffatome, insbesondere 12 bis 20 Kohlenstoffatome auf. Bevorzugt sind die Verbindungen Sorbitan Palmitat und Sorbitan Stearat enthalten, besonders bevorzugt ist Sorbitan Stearat enthalten. Sorbitan Stearat ist beispielsweise erhältlich unter der Handelsbezeichnung Tego SMS MB von der Firma Evonik.

In der erfindungsgemäßen Emulsion ist der wenigstens eine Sorbitanfettsäureester mit einem Gesamtgehalt von 0,1 bis 6,0 Gew-%, bevorzugt von 2,0 bis 4,0 Gew.-% enthalten, bezogen auf das Gesamtgewicht der Emulsion.

Die erfindungsgemäße Emulsion enthält wenigstens ein Alkylphosphat. Alkylphosphate werden bevorzugt als Mono- oder Dialkylphosphate eingesetzt. Insbesondere bevorzugt sind Monoalkylphosphate. Die Alkylreste weisen von 6 bis 22 Kohlenstoffatome, insbesondere 8 bis 18 Kohlenstoffatome auf. Die Alkylphosphate, insbesondere Monoalkylphosphate, liegen bevorzugt in Form ihrer Natrium- oder Kaliumsalze, weiter bevorzugt als Kaliumsalze vor. Noch weiter bevorzugt sind die Verbindungen Kalium Cetylphosphat und Kalium Stearylphosphat, am meisten bevorzugt ist die Verbindung Kalium Cetyl Phosphat, beispielsweise erhältlich unter Handelsbezeichnung Emulsiphos von der Firma Symrise.

In der erfindungsgemäßen Emulsion ist das wenigstens eine Alkylphosphat mit einem Gesamtgehalt von 0,1 bis 4,0 Gew-%, bevorzugt von 0,5 bis 2,5 Gew.-% enthalten, bezogen auf das Gesamtgewicht der Emulsion.

Erfindungsgemäß vorteilhaft ist die Kombination von Sorbitan Stearat und Kalium Cetyl Phosphat in der Emulsion enthalten. Weiter vorteilhaft ist es, wenn, in Bezug auf die Emulgatoren, nur Sorbitan Stearat und Kalium Cetyl Phosphat in der Emulsion enthalten sind.

In der erfindungsgemäßen Emulsion ist eine Kombination von wenigstens zwei Emollentien enthalten. Unter Emollientien werden Substanzen verstanden, die das Haar weich und geschmeidig machen können. Es handelt sich dabei vorteilhaft um lipophile Komponenten.

Erfindungsgemäß vorteilhaft sind die Emollientien Ester des Glycerins, also Acylglyceride, die als Mono-, Di- und Triglyceride vorliegen können. Besonders vorteilhaft sind Ester des Glycerins mit Fettsäuren, die ausgehend von natürlichen Ölen, wie beispielsweise Kokosöl, Palmöl, Sojaöl und andere mehr gewonnen werden. Derartige Fettsäuren sind jeweils Mischungen, die die Zusammensetzung der jeweiligen natürlichen Öle widerspiegeln. Diese Mischungen enthalten also auch Fettsäuren mit Doppelbindungen. Wenn in einem Hydrierungsschritt diese Doppelbindungen hydriert (englisch: hydrogenated) werden, werden hydrierte Verbindungen erhalten, die ganz besonders vorteilhaft sind; im Folgenden als hydrierte, auf natürlichen Ölen-basierte Acylglyceride bezeichnet. Erfindungsgemäß weiter ganz besonders vorteilhaft werden die Verbindungen Hydrogenated Coco Glycerides, Hydrogenated Soy Glyceride und/oder Hydrogenated Palm Glyceride eingesetzt, die Verbindung Hydrogenated Coco Glycerides ist am meisten bevorzugt. Hydrogenated Coco Glycerides ist beispielsweise unter dem Handelsnamen Cutina BD bei der Firma BASF erhältlich.

In der erfindungsgemäßen Emulsion ist das wenigstens eine Acylglycerid mit einem Gesamtgehalt von 0,1 bis 8,0 Gew-%, bevorzugt von 2,0 bis 7,0 Gew.-% enthalten, bezogen auf das Gesamtgewicht der Emulsion.

Ebenso vorteilhaft sind Esteröle, erfindungsgemäß wenigstens ein Esteröl, das wenigstens 12 Kohlenstoffatome und wenigstens eine Verzweigung aufweist.

Im allgemeinen sind Esteröle Ester von gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen ausgewählt werden. Trotz des Begriffs Esteröl können diese Substanzen flüssig oder fest sein.

Erfindungsgemäß vorteilhaft sind Esteröle, die Ester von verzweigten, primären Alkoholen mit 6 bis 22 Kohlenstoffatomen mit Fettsäuren, die 6 bis 22 Kohlenstoffatome aufweisen, sind. Weiter vorteilhaft sind die Esteröle bei Raumtemperatur (25°C) fest. Ganz besonders vorteilhafte Esteröle sind Ethylhexyl Palmitat und Ethylhexyl Stearat, die jeweils unter den Handelsnamen Radia 7722 MB bei der Firma Oleon N.V. und Cetiol 868 bei der Firma BASF erhältlich sind.

In der erfindungsgemäßen Emulsion ist das wenigstens eine Esteröl aufweisend wenigstens 12 Kohlenstoffatome und wenigstens eine Verzweigung mit einem Gesamtgehalt von 0,01 bis 4,0 Gew-%, bevorzugt von 0,1 bis 2,0 Gew.-% enthalten, bezogen auf das Gesamtgewicht der Emulsion.

Erfindungsgemäß vorteilhaft ist die Kombination von Hydrogenated Coco Glycerides und Ethylhexyl Stearat in der Emulsion enthalten. Weiter vorteilhaft ist es, wenn, in Bezug auf die Emollientien, nur Hydrogenated Coco Glycerides und Ethylhexyl Stearat in der Emulsion enthalten sind.

Um einen Styling-Effekt zu erzielen, ist die Einarbeitung eines festigenden und/oder Film-bildenden Polymers erforderlich. Derartige Polymere wechselwirken mit der Haaroberfläche und bilden einen Film aus. Zwischen einzelnen Haaren oder Haarsträhnen können dann kleine Brücken aus Polymeren ausgebildet werden, die wesentlich dazu beitragen, dass eine Frisur fixiert wird. Üblicherweise sind diese Polymere synthetischer Natur und daher kaum oder nur schlecht biologisch abbaubar. Die Verbraucher wünschen aber zunehmend Produkte, die Inhaltsstoffe enthalten, die gut biologisch abbaubar sind und die Umwelt nicht belasten. Um diesem Wunsch gerecht zu werden, ist ein Film-bildendes/festigendes Polymer enthalten, das Natur-basiert ist. Bevorzugt ist Distärkephosphat enthalten. Dabei handelt es sich um eine modifizierte Stärke, die ausgehend von natürlicher Stärke oder abgebauter Stärke beispielsweise durch Veresterung mit Natriumtrimetaphosphat oder Phosphoroxychlorid erhalten werden kann.

Distärkephosphat kann beispielsweise unter dem Handelsnamen Agenajel 20.306 von der Firma Agrana erhalten werden.

In der erfindungsgemäßen Emulsion ist das wenigstens eine Film-bildendes/festigendes Polymer, insbesondere Distärkephosphat, mit einem Gesamtgehalt von 0,01 bis 4,0 Gew-%, bevorzugt von 0,1 bis 3,0 Gew.-% enthalten, bezogen auf das Gesamtgewicht der Emulsion.

In der erfindungsgemäßen Emulsion kann zusätzlich wenigstens ein Moisturizer enthalten sein. Als Moisturizer werden Stoffe oder Stoffgemische bezeichnet, welche kosmetischen Zubereitungen die Eigenschaft verleihen, nach dem Auftragen bzw. Verteilen auf der Haaroberfläche die Feuchtigkeitsabgabe zu reduzieren und/oder die Hydratation der Haaroberfläche bzw. der tiefer liegenden Schichten positiv zu beeinflussen. Vorteilhafte Moisturizer im Sinne der vorliegenden Erfindung sind beispielsweise Glycerin, Sorbitol, Butylenglykol, Propylenglykol, Caprylylglycol, Milchsäure und/oder Lactate, insbesondere Natriumlactat, Biosaccaride Gum-1, Glycine Soja, Ethylhexylglycerin, Pyrrolidoncarbonsäure und Harnstoff. Bevorzugt wird der wenigstens eine Moisturizer aus der Gruppe Glycerin, Sorbitol, Butylenglykol, Propylenglykol, Caprylylglycol, Milchsäure und/oder Salze der Milchsäure ausgewählt. Es können auch zwei oder mehr verschiedene Moisturizer enthalten sind, beispielsweise Sorbitol, Caprylylglycol und Glycerin.

Wenn in der erfindungsgemäßen Emulsion wenigstens ein Moisturizer enthalten ist, so liegt er mit einem Gesamtgehalt von 0,1 bis 20 Gew. %, bevorzugt 0,2 bis 15,0 Gew. %, bezogen auf das Gesamtgewicht der Zubereitung, vor.

In der erfindungsgemäßen Emulsion kann wenigstens eine Substanz enthalten sein, die eine stabilisierende Wirkung auf die Zubereitung entfaltet. Derartige Substanzen werden im Folgenden als stabilisierende Substanzen bezeichnet. Diese Substanzen sind nicht in der Liste der zugelassenen Konservierungsstoffe (Kosmetikverordnung Anlage 6; Verordnung (EG) Nr. 1223/2009, Anhang V) aufgeführt, gleichwohl haben sie aber eine stabilisierende Wirkung auf die jeweiligen kosmetischen Zubereitungen. Derartige Substanzen können aus der Gruppe Propylenglycol, Ethylhexylglycerin, 1,2-Hexanediol, Methylpropanediol, Butylenglycol, Caprylylglycol, Decylenglycol, Hydroxyacetophenone und/oder Pentylenglycol ausgewählt werden. Wie aus der Auflistung der Substanzen ersichtlich, die als Moisturizer wirken können, gibt es eine Überlappung mit den Substanzen mit stabilisierender Wirkung. Die Substanzen Butylenglycol, Caprylylglycol, Propylenglycol und Ethylhexylglycerin wirken als Moisturizer und gleichzeitig entfalten sie eine stabilisierende Wirkung in Bezug auf die jeweilige Zubereitung. Erfindungsgemäß vorteilhaft ist es Hydroxyacetophenone als stabilisierende Substanz einzusetzen.

Wenn in der erfindungsgemäßen Emulsion wenigstens eine Substanz mit stabilisierender Wirkung enthalten ist, so liegt sie mit einem Gesamtgehalt von 0,1 bis 20 Gew. %, bevorzugt 5,0 bis 15,0 Gew.-% in der erfindungsgemäßen Zubereitung vor, bezogen auf das Gesamtgewicht der Zubereitung.

In der erfindungsgemäßen Emulsion kann wenigstens ein Fettalkohol enthalten sein. Fettalkohole sind aliphatische, langkettige, einwertige, meist primäre Alkohole. Der Begriff Fettalkohol wird in der Regel für Fettalkohole mit einer Kettenlänge von 6 bis 22 Kohlenstoffatomen verwendet. Die Kohlenstoffkette der Fettalkohole kann eine oder mehr Doppelbindungen aufweisen und/oder unverzweigt oder verzweigt sein. Erfindungsgemäß bevorzugt werden gesättigte, unverzweigte Fettalkohole ausgewählt, besonders bevorzugt Cetylalkohol und/oder Myristylalkohol.

Wenn in dem erfindungsgemäßen Creme Gel wenigstens ein Fettalkohol enthalten ist, so liegt der wenigstens eine Fettalkohol mit einem Gesamtgehalt von 0,1 bis 15,0 Gew. %, bevorzugt 3,0 bis 10,0 Gew. % vor, bezogen auf das Gesamtgewicht der Zubereitung.

Erfindungsgemäß vorteilhaft ist ebenfalls die Verwendung einer oder mehrerer Substanzen der Vitamine der B Gruppe und/oder des Vitamin B-Komplexes, die den erfindungsgemäßen Zusammensetzungen zusätzlich zugesetzt werden können. Substanzen der Vitamin B Gruppe und/oder des Vitamin B-Komplexes sind üblicherweise wasserlöslich und spielen insbesondere für den Zellmetabolismus bei Pflanzen und Tieren eine besondere Rolle.

Beispiele erfindungsgemäßer Substanzen der Vitamine der B Gruppe und/oder des Vitamin B-Komplex sind unter anderem Thiamin (Vitamin B1), Riboflavin (Vitamin B2), Nicotinsäure (Vitamin B3), Nicotinsäureamid (Niacinamid), Pantothensäure (Vitamin B5), Panthenol (Provitamin B5), Panthenoltriacetat, Panthenolmonoethylether, Pantolacton, Pyridoxin und Pyridoxal.

Bevorzugt ist die Verwendung von Panthenol als Substanz der Vitamine der B Gruppe und/oder des Vitamin B-Komplexes in den erfindungsgemäßen Zusammensetzungen.

Panthenol kann beispielsweise unter dem Handelsnamen D-Panthenol 75 W als 77 % Lösung in Wasser von der BASF bezogen werden.

Eine oder mehrere Substanzen der Vitamine der B Gruppe und/oder des Vitamin B-Komplex sind erfindungsgemäß in einem Anteil von 0,005 Gew.-% bis 5 Gew.-%, bevorzugt 0,05 Gew.-% bis 2 Gew.-%, insbesondere bevorzugt 0,01 Gew.-% bis 1,0 Gew.-%, bezogen auf das Gesamtgewicht der Emulsion und den Aktivgehalt, in den erfindungsgemäßen Zusammensetzungen enthalten.

Der Begriff "natürlich" im Zusammenhang mit natürlichen Inhaltsstoffen für Kosmetika ist in der ISO 16128 festgelegt. Gemäß dieser Richtlinie kann man natürliche, kosmetische Inhaltsstoffe zusammenfassend als Stoffe beschreiben, die von Pflanzen, Tieren, Mineralien oder Mikroorganismen erhältlich sind, eingeschlossen sind auch solche Stoffe, die von den genannten Quellen durch physikalische Prozesse, Fermentationsprozesse (nur solche Fermentationsprozesse, die auch in der Natur ablaufen und die zu Produkten führen, die auch in der Natur entstehen) und andere Herstellungsmethoden ohne beabsichtigte chemische Modifikation erhalten werden. Die Mikroorganismen dürfen nur solche sein, die nicht gentechnisch modifiziert wurden.

Im Sinne der vorliegenden Erfindung sind Natur-basierte Substanzen, insbesondere Natur-basierte Polymere, Verbindungen, die von natürlichen Polymeren abgeleitet sind, beispielsweise durch chemische Modifizierungen.

Der Begriff der biologischen Abbaubarkeit beschreibt den Prozess des Abbaus von organischen Verbindungen durch Lebewesen, insbesondere Saprobionten. Im Idealfall entstehen anorganische Stoffe, wie beispielsweise $CO_2$, $O_2$ und Ammoniak; Verbindungen, die von Pflanzen und Mikroorganismen wieder für den Aufbau von organischen Verbindungen genutzt werden.

Organische, chemische Verbindungen, die leicht biologisch abbaubar sind, sind nach OECD 301, für kosmetische Zusammensetzungen meist nach OECD 301 B klassifiziert. Diese Substanzen oder Zusammensetzungen können rasch und vollständig abgebaut werden.

Organische, chemische Verbindungen, die nach OECD 302 klassifiziert sind, sind zwar eingeschränkt, jedoch grundsätzlich biologisch abbaubar.

Der erfindungsgemäßen Emulsion werden keine Silikon-Verbindungen zugesetzt, ebenso sind keine Silikon-Verbindungen enthalten, die mit an sich anderen Rohstoffen in die Zubereitung gelangen. Die erfindungsgemäße Emulsion ist also frei von Silikon-Verbindungen. Silikon-Verbindungen zeichnen sich durch das Vorhandensein von Silicium- und Sauerstoffatomen aus, die miteinander verknüpft sind und eine Vielzahl verschiedener Verbindungen ausbilden können.

Weiterhin werden der erfindungsgemäßen Emulsion keine PEG-haltigen Verbindungen zugesetzt, ebenso sind keine PEG-haltigen Verbindungen enthalten, die mit an sich anderen Rohstoffen in die Zubereitung gelangen. Die erfindungsgemäße Emulsion ist also frei von PEG-haltigen-Verbindungen. PEG ist die Abkürzung für Polyethylenglycol; viele der Struktureinheiten ($-CH_2-CH_2-O-$) sind dabei miteinander verknüpft. Diese Strukturen können bewirken, dass eine Penetration durch die Haut bewirkt oder verstärkt wird. Unter PEG-haltige Verbindungen werden Polyethylenglykole und Polyethylenglykolderivate verstanden.

Ebenso werden der Emulsion keine synthetischen Polymere zugesetzt, auch sind keine synthetischen Polymere enthalten, die mit an sich anderen Rohstoffen in die Zubereitung gelangen. Polymere Verbindungen sind synthetische Verbindungen, die sich aus einem oder verschiedenen Monomeren zusammensetzen. Diese Monomere sind in der Regel rein synthetischer Natur sein. Ebenso kann das gesamte Polymer synthetischer Natur sein. Derartige Polymere werden nicht von lebenden Organismen in biologischen Prozessen hergestellt. Die erfindungsgemäße Emulsion ist frei von synthetischen Polymeren.

Ebenso Gegenstand der vorliegenden Erfindung ist die Verwendung der Kombination von Hydrogenated Coco Glycerides und Ethylhexyl Palmitat und/oder Ethylhexyl Stearat enthalten in einer Emulsion, insbesondere in einer Emulsion vorgesehen für eine Haar-kosmetische Anwendung, weiter insbesondere in einem Creme Gel, enthaltend

- eine Kombination von Emulgatoren umfassend wenigstens einen Sorbitanfettsäureester und wenigstens ein Alkylphosphat und
- eine Kombination von wenigstens zwei Emollentien, umfassend wenigstens ein Acylglycerin und wenigstens ein Esteröl, wobei das Esteröl wenigstens 12 Kohlenstoffatome und wenigstens eine Verzweigung aufweist,

wobei die Emulsion frei ist von

- PEG und PEG-haltigen Verbindungen,
- Silikonverbindungen,
- Verbindungen fossilen Ursprungs und Verbindungen, die Komponenten enthalten, die fossilen Ursprungs sind und
- synthetischen Polymeren,

zur Pflege der Haare.

Um die die Pflegeleistung eines beispielhaften erfindungsgemäßen Produktes zu evaluieren, wurde ein In Use Test in Form eines monadischen Tests durchgeführt. Hierbei erhält jeder Teilnehmer jeweils ein Produkt, und zwar erhielt eine Gruppe von Teilnehmern das Produkt C10 (Nivea Pflege & Halt Creme Gel Regenerierend) und die zweite Gruppe das Produkt K30 (siehe Beispielrezepturen, gemäß Beispiel 1). Der Anwendungszeitraum betrug eine Woche, anschließend wurde mittels eines Online-Fragebogens eine Produkt-Bewertung durchgeführt. Die Teilnehmerinnen waren alle weiblich, im Alter von 18 bis 60 Jahren.

Eine Evaluierung der Pflegeleistung des jeweiligen Testproduktes erfolgte anhand folgender Aussagen (Angabe in % der Zustimmung):

|  | C10 | K30 |
|---|---|---|
| Base (100%) n= | 80 | 77 |
|  |  |  |
| *"yes" in %* |  |  |
| My hair feels cared for | 88 | 97 |

(fortgesetzt)

| *"yes" in* % | | |
|---|---|---|
| My hair shines naturally | 84 | 89 |
| The cream gel provides shine for my hair | 85 | 87 |
| The cream gel does not dry out my hair | 89 | 98 |
| My hair looks healthy | 88 | 95 |
| My hair feels healthy | 88 | 92 |
| My hair feels supple | 89 | 98 |

Aus dieser Übersicht wird deutlich, dass dem erfindungsgemäßen Produkt die abgefragten Eigenschaften zugeordnet wurden, und zwar in einem höheren Maße als bei dem Kontrollprodukt. Damit ist belegt, dass dem untersuchten Beispiel des erfindungsgemäßen Produktes (K30) die abgefragten Eigenschaften zugeordnet werden. Somit sind von den Anwenderinnen die Attribute hinsichtlich einer Pflegeleistung dem beispielhaften erfindungsgemäßen Produkt häufiger zugeordnet worden als dem Vergleichsprodukt.

**Beispiele:**

Die untenstehenden Beispiele sollen die Erfindung verdeutlichen, ohne sie einzuschränken.
Die Mengenangaben beziehen sich auf Gew.-%, sofern nicht anders angegeben.

| | 1 | 2 | 3 | 4 | 5 | 6 |
|---|---|---|---|---|---|---|
| EU-INCI | | | | | | |
| Aqua | Add 100 | Add 100 | Add 100 | Add 100 | Add 100 | Add 100 |
| Myristyl Alcohol | 3,3 | 3,25 | 2,8 | 3 | 3,5 | 3,5 |
| Cetyl Alcohol | 4 | 4,5 | 3,75 | 4 | 4,5 | 4,5 |
| Hydrogenated Coco-Glycerides | 6 | 5,5 | 6,25 | 6 | 5 | 5 |
| Sorbitan Stearate | 3 | 3,5 | | 2,8 | | 3 |
| Sorbitan Palmitate | | | 3,5 | | 3 | |
| Potassium Cetyl Phosphate | 1,5 | 1,08 | 1,02 | 1,32 | 1,5 | 1,5 |
| Hydrogenated Palm Glycerides | 1 | 0,72 | 0,58 | 0,88 | 1 | 1 |
| Ethylhexyl Stearate | 0,6 | | | | | 0,5 |
| Ethylhexyl Cocoate | | 0,2 | 0,7 | | | |
| Ethylhexyl Palmitate | | 0,4 | | 0,5 | 0,6 | |
| Distarch Phosphate | 1,2 | 1,5 | 0,8 | 1 | 1 | 1,2 |
| Hydroxyacetophenone | 0,4 | 0,4 | 0,4 | 0,4 | 0,4 | 0,4 |
| Caprylyl Glycol | 0,2 | 0,2 | 0,2 | 0,2 | 0,2 | 0,2 |
| Sorbitol | 13 | 12 | 13 | 11 | 11 | 12 |
| Tapioca Starch | 0,2 | 0,15 | 0,25 | 0,2 | 0,2 | 0,25 |
| Parfum | 0,4 | 0,4 | 0,4 | 0,4 | 0,4 | 0,4 |

**Patentansprüche**

1. Emulsion, insbesondere eine Emulsion vorgesehen für eine Haar-kosmetische Anwendung, weiter insbesondere ein Creme Gel, enthaltend

- eine Kombination von Emulgatoren, umfassend wenigstens einen Sorbitanfettsäureester und wenigstens ein Alkylphosphat und
- eine Kombination von wenigstens zwei Emollentien, umfassend wenigstens ein Acylglycerin und wenigstens ein Esteröl, wobei das Esteröl wenigstens 12 Kohlenstoffatome und wenigstens eine Verzweigung aufweist,

wobei die Emulsion frei ist von

- PEG und PEG-haltigen Verbindungen,
- Silikonverbindungen,
- Verbindungen fossilen Ursprungs und Verbindungen, die Komponenten enthalten, die fossilen Ursprungs sind und
- synthetischen Polymeren.

2. Emulsion nach Anspruch 1, **dadurch gekennzeichnet, dass** die Emulsion eine wässrige Zubereitung ist mit einem Wassergehalt von 50 bis 80 Gew.-%, bevorzugt 55 bis 70 Gew.-%, bezogen auf das Gesamtgewicht der Zubereitung.

3. Emulsion nach Anspruch 1 und/oder 2, **dadurch gekennzeichnet, dass** die Emulsion eine O/W-Emulsion ist.

4. Emulsion nach wenigstens einem Anspruch der vorstehenden Ansprüche, **dadurch gekennzeichnet, dass** der wenigstens eine Sorbitanfettsäureester Fettsäurereste mit 6 bis 22 Kohlenstoffatome, insbesondere 12 bis 20 Kohlenstoffatome aufweist, bevorzugt ist Sorbitan Stearat enthalten.

5. Emulsion nach wenigstens einem Anspruch der vorstehenden Ansprüche, **dadurch gekennzeichnet**, das der wenigstens eine Sorbitanfettsäureester mit einem Gesamtgehalt von 0,1 bis 6,0 Gew-%, bevorzugt von 2,0 bis 4,0 Gew.-% enthalten ist, bezogen auf das Gesamtgewicht der Emulsion.

6. Emulsion nach wenigstens einem der vorstehenden Ansprüche, **dadurch gekennzeichnet, dass** wenigstens ein Monoalkylphosphat enthalten ist, bevorzugt wenigstens ein Monoalkylphosphat aufweisend Alkylreste weisen mit 6 bis 22 Kohlenstoffatomen, insbesondere 8 bis 18 Kohlenstoffatomen, weiter bevorzugt wenigstens ein Monoalkylphosphat aufweisend Alkylreste weisen mit 8 bis 18 Kohlenstoffatomen und in Form des Natrium- oder Kaliumsalze, am meisten bevorzugt Kalium Cetyl Phosphat.

7. Emulsion nach wenigstens einem der vorstehenden Ansprüche, **dadurch gekennzeichnet, dass** das wenigstens eine Alkylphosphat mit einem Gesamtgehalt von 0,1 bis 4,0 Gew-%, bevorzugt von 0,5 bis 2,5 Gew.-% enthalten ist, bezogen auf das Gesamtgewicht der Emulsion.

8. Emulsion nach wenigstens einem der vorstehenden Ansprüche, **dadurch gekennzeichnet, dass** die Kombination von Sorbitan Stearat und Kalium Cetyl Phosphat enthalten ist.

9. Emulsion nach wenigstens einem der vorstehenden Ansprüche, **dadurch gekennzeichnet, dass** das wenigstens eine Acylglycerin ein Ester des Glycerins mit Fettsäuren, gewonnen aus natürlichen Ölen, insbesondere aus Kokosöl, Palmöl und Sojaöl ist, bevorzugt hydrierte, auf natürlichen Ölen-basierte Acylglyceride, ganz besonders bevorzugt Hydrogenated Coco Glycerides.

10. Emulsion nach wenigstens einem der vorstehenden Ansprüche, **dadurch gekennzeichnet, dass** das wenigstens eine Acylglycerid mit einem Gesamtgehalt von 0,1 bis 8,0 Gew-%, bevorzugt von 2,0 bis 7,0 Gew.-% enthalten ist, bezogen auf das Gesamtgewicht der Emulsion.

11. Emulsion nach wenigstens einem der vorstehenden Ansprüche, **dadurch gekennzeichnet, dass** das wenigstens eine Esteröl, aufweisend wenigstens 12 Kohlenstoffatome und wenigstens eine Verzweigung ausgewählt wird aus Ethylhexyl Palmitat und/oder Ethylhexyl Stearat.

12. Emulsion nach wenigstens einem vorstehenden der Ansprüche, **dadurch gekennzeichnet, dass** das wenigstens eine Esteröl aufweisend wenigstens 12 Kohlenstoffatome und wenigstens eine Verzweigung mit einem Gesamtgehalt von 0,01 bis 4,0 Gew-%, bevorzugt von 0,1 bis 2,0 Gew.-% enthalten ist, bezogen auf das Gesamtgewicht der Emulsion.

13. Emulsion nach wenigstens einem der vorstehenden Ansprüche, **dadurch gekennzeichnet, dass** die Kombination von Hydrogenated Coco Glycerides und Ethylhexyl Palmitat und/oder Ethylhexyl Stearat enthalten ist.

14. Emulsion nach wenigstens einem vorstehenden der Ansprüche, **dadurch gekennzeichnet, dass** wenigstens ein Film-bildendes/festigendes Polymer enthalten ist, das Natur-basiert ist, bevorzugt Distärkephosphat.

15. Emulsion nach wenigstens einem vorstehenden der Ansprüche, **dadurch gekennzeichnet, dass** das wenigstens ein Natur-basiertes Film-bildendes/festigendes Polymer, insbesondere Distärkephosphat, mit einem Gesamtgehalt von 0,01 bis 4,0 Gew-%, bevorzugt von 0,1 bis 3,0 Gew.-% enthalten ist, bezogen auf das Gesamtgewicht der Emulsion.

16. Verwendung der Kombination von Hydrogenated Coco Glycerides und Ethylhexyl Palmitat und/oder Ethylhexyl Stearat, enthalten in einer Emulsion, insbesondere in einer Emulsion vorgesehen für eine Haar-kosmetische Anwendung, weiter insbesondere in einem Creme Gel, gemäß wenigstens einem der vorstehenden Ansprüche, zur Pflege der Haare.

EUROPÄISCHER RECHERCHENBERICHT

Nummer der Anmeldung

EP 22 21 1454

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2019/072375 A1 (SYMRISE AG [DE]) 18. April 2019 (2019-04-18) | 1-16 | INV. A61K8/37 |
| Y | * Seiten 2-3, Absatz 12-14; Beispiel 2; Tabelle 2 * * Seiten 9-10, Absatz 31-34 * * Seiten 34-35, Absatz 142-143 * ----- | 1-16 | A61K8/49 A61K8/55 A61Q5/12 |
| Y | DATABASE GNPD [Online] MINTEL; 24. Januar 2022 (2022-01-24), anonymous: "Styling Putty", XP093058693, Database accession no. 9226012 * Zusammenfassung * ----- | 1-16 | |
| Y | JP 2020 094035 A (MANDOM CORP) 18. Juni 2020 (2020-06-18) * Seite 1, Absatz 5-11 * * Seiten 3-4, Absatz 33-34 * * Seiten 4-5, Absatz 41-45 * * Seiten 5-6, Absatz 49 * ----- | 1-16 | |
| Y | WO 2020/043365 A1 (BEIERSDORF AG [DE]) 5. März 2020 (2020-03-05) | 1-15 | RECHERCHIERTE SACHGEBIETE (IPC) A61K |
| A | * Seite 2, Zeile 20 - Seite 3, Zeile 6 * * Seite 4, Zeilen 9-18 * * Seite 9, Zeile 32 - Seite 10, Zeile 4 * * Seite 12, Zeilen 13-17 * ----- | 16 | A61Q |
| Y | FR 3 102 059 A1 (ALGOLOGIE [FR]) 23. April 2021 (2021-04-23) | 1-15 | |
| A | * Beispiel 1 * * Seiten 14-15, Absätze 102,105-106 * ----- | 16 | |
| Y | CN 106 511 210 B (NOX BELLCOW COSMETICS CO LTD) 25. Oktober 2019 (2019-10-25) | 1-15 | |
| A | * Beispiele * * Seite 1, Absätze 4,6 * ----- | 16 | |

-/--

Der vorliegende Recherchenbericht wurde für alle Patentansprüche erstellt

| Recherchenort | Abschlußdatum der Recherche | Prüfer |
|---|---|---|
| München | 10. Juli 2023 | Ruckebusch, Virginie |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes Dokument

EPO FORM 1503 03.82 (P04C03)

Europäisches
Patentamt

European
Patent Office

Office européen
des brevets

**EUROPÄISCHER RECHERCHENBERICHT**

Nummer der Anmeldung

**EP 22 21 1454**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 4 699 778 A (MARTY JEAN-PIERRE [FR]) 13. Oktober 1987 (1987-10-13) | 1-15 | |
| A | * Beispiel 2 * | 16 | |
| | ----- | | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| **München** | **10. Juli 2023** | **Ruckebusch, Virginie** |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes
Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 22 21 1454

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-07-2023

| Im Recherchenbericht angeführtes Patentdokument | Datum der Veröffentlichung | Mitglied(er) der Patentfamilie | Datum der Veröffentlichung |
|---|---|---|---|
| WO 2019072375 A1 | 18-04-2019 | BR 112020006732 A2 | 06-10-2020 |
| | | CN 111194206 A | 22-05-2020 |
| | | EP 3694474 A1 | 19-08-2020 |
| | | JP 7104149 B2 | 20-07-2022 |
| | | JP 2021501745 A | 21-01-2021 |
| | | KR 20200067867 A | 12-06-2020 |
| | | US 2023134686 A1 | 04-05-2023 |
| | | WO 2019072375 A1 | 18-04-2019 |
| JP 2020094035 A | 18-06-2020 | KEINE | |
| WO 2020043365 A1 | 05-03-2020 | BR 112021003000 A2 | 11-05-2021 |
| | | CN 112437655 A | 02-03-2021 |
| | | DE 102018214478 A1 | 05-03-2020 |
| | | EP 3843691 A1 | 07-07-2021 |
| | | US 2021338559 A1 | 04-11-2021 |
| | | WO 2020043365 A1 | 05-03-2020 |
| FR 3102059 A1 | 23-04-2021 | KEINE | |
| CN 106511210 B | 25-10-2019 | KEINE | |
| US 4699778 A | 13-10-1987 | BE 902280 A | 25-10-1985 |
| | | CA 1266831 A | 20-03-1990 |
| | | CH 664490 A5 | 15-03-1988 |
| | | DE 3515231 A1 | 17-04-1986 |
| | | FR 2563431 A1 | 31-10-1985 |
| | | GB 2157566 A | 30-10-1985 |
| | | IT 1181883 B | 30-09-1987 |
| | | JP H0519527 B2 | 17-03-1993 |
| | | JP S6127912 A | 07-02-1986 |
| | | OA 08003 A | 31-01-1987 |
| | | US 4699778 A | 13-10-1987 |
| | | ZA 852958 B | 25-06-1986 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2909087 C2